(19) Europäisches Patentamt
European Patent Office
Office européen des brevets

(11) Veröffentlichungsnummer: **0 275 014 B1**

# (12) EUROPÄISCHE PATENTSCHRIFT

(45) Veröffentlichungstag der Patentschrift: **27.06.90**

(51) Int. Cl.⁵: **C07D 487/04**, C08F 4/00 // (C07D487/04, 249:00, 239:00)

(21) Anmeldenummer: **88100034.3**

(22) Anmeldetag: **05.01.88**

## (54) Neue Triazolopyrimidine und ihre Verwendung als Initiatoren.

(30) Priorität: **16.01.87 DE 3701065**
**28.01.87 DE 3702392**

(43) Veröffentlichungstag der Anmeldung: **20.07.88 Patentblatt 88/29**

(45) Bekanntmachung des Hinweises auf die Patenterteilung: **27.06.90 Patentblatt 90/26**

(84) Benannte Vertragsstaaten: **DE ES FR GB IT NL**

(56) Entgegenhaltungen:
**BULLETIN OF THE CHEMICAL SOCIETY OF JAPAN, Band 54, 1981 C. YAMAZAKI; "Cyclization of Isothiosemicarbazones. IV. Synthesis of th (1,2,4)triazolo-(1,5-c)pyrimidine Ring System" Seiten 1767-1772**
**THE JOURNAL OF ORGANIC CHEMISTRY, Band 50, 1985 C. YAMAZAKI; "Cyclization of Isothiosemicarbazones. 5. (1,2,4)-Triazolo(1,5-c)pyrimidines" Seiten 3956-3959**
**THE JOURNAL OF ORGANIC CHEMISTRY, C. YAMAZAKI et al.; "Cyclization of Isothiosemicarbazones. 6. The Formation and Structures of N-Alkenyl-1,2,4-triazoles and Related Compounds" Seiten 5513-5516**
**CHEMICAL ABSTRACTS, Band 105, Nr. 11, 15.**

(73) Patentinhaber: **BAYER AG, D-5090 Leverkusen 1 Bayerwerk(DE)**

(72) Erfinder: **Lindner, Christian, Dr., Riehler Strasse 200, D-5000 Köln 60(DE)**
Erfinder: **Dickoré, Karlfried, Dr., Nicolai-Hartmann-Strasse 19, D-5090 Leverkusen(DE)**
Erfinder: **Süling, Carlhans, Dr., Carl-Leverkus-Strasse 10, D-5068 Odenthal(DE)**
Erfinder: **Korte, Siegfried, Dr., Engstenberger Höhe 3, D-5068 Odenthal(DE)**
Erfinder: **Podszun, Wolfgang, Dr., Wolfskaul 4, D-5000 Köln 80(DE)**

(56) Entgegenhaltungen: (Fortsetzung)
**September 1986 Y, MIYAMOTO; YOSHIKO; "Synthesis of (1,2,4)triazolo(1,5-c)pyrimidine derivatives" Seite 630, Spalte 2, Zusammenfassung Nr. 97 409n**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 275 014 B1

**Beschreibung**

Gegenstand der Erfindung sind neue Triazolopyrimidine der Formel I

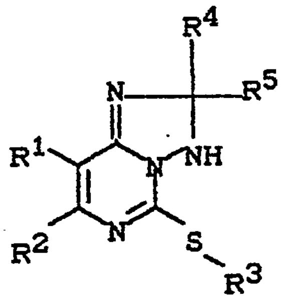

I

in welcher

$R^1$ = CN, Alkylsulfonyl, Arylsulfonyl, Aryl
$R^2$ = H, Alkyl, Alkylthio
$R^3$ = Alkyl, (gegebenenfalls substituiertes) Aralkyl, Alkenyl, Alkinyl
$R^4$ = Alkyl, Aralkyl, Aryl
$R^5$ = Alkyl, Aralkyl, Aryl oder
$R^4$ und $R^5$ zusammen mit dem Kohlenstoffatom einen (gegebenenfalls substituierten oder überbrückten) carbocyclischen 5-, 6- oder 7-Ring
bedeuten,
worin $R^4$ nicht Methyl oder Aryl sein kann, wenn $R^1$ für CN, $R^2$ für H, $R^3$ für Methyl und $R^5$ für Methyl oder Aryl steht und worin Alkyl vorzugsweise solche mit 1–9 C-Atomen und Aryl vorzugsweise solche mit 6–12 C-Atomen sind.

Ein weiterer Gegenstand der Erfindung ist ein Verfahren zur Herstellung dieser Verbindungen und ihre Verwendung als Initiatoren für die radikalische Polymerisation.

Die radikalische Polymerisation von α,β-ungesättigten Verbindungen ist eine weitverbreitete Methode zur Herstellung verschiedenster Polymerisate. Solche Polymerisationen werden üblicherweise mit sogenannten Initiatoren katalysiert, als solche sind z.B. zu nennen: Peroxide, Azoverbindungen, Perester, Hydroperoxide, Persulfate, Perphosphate. Die Initiierung mit solchen Verbindungen kann auch bekanntlich nach der Redoxvariante erfolgen; dabei stellt ein Redoxpaar aus wenigstens zwei Verbindungen ein wirksames Initiierungssystem dar.

Die Wirksamkeit der meisten Initiatoren kann aber von der Sauerstoffkonzentration am Polymerisationsort (Reaktor etc.) beeinflußt werden. Um diesen Einfluß zu reduzieren, wird häufig unter Stickstoff polymerisiert oder der Sauerstoff durch bestimmte gezielte Überdosierung des Initiators kompensiert. Meistens ist der Sauerstoffeinfluß bei der technischen Polymerisation schwierig kontrollierbar, insbesondere bei einer Vergrößerung der Polymerisationsansätze. Eine gleichmäßige, reproduzierbare Initiierung ist aber erforderlich, um hochwertige Polymerisate mit gleichmäßigen physikalischen Eigenschaften herzustellen.

Die Verbindungen der Formel I sind als Initiatoren für die radikalische Polymerisation unempfindlich gegen Sauerstoff.

Gegenstand der Erfindung ist somit die Verwendung von Triazolopyrimidinen der Formel (I)

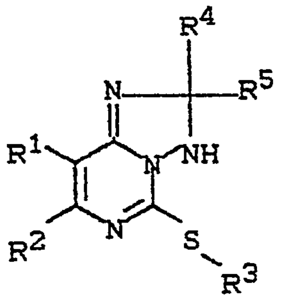

I

in welcher

$R^1$ = CN, Alkylsulfonyl, Arylsulfonyl, Aryl
$R^2$ = H, Alkyl, Alkylthio

$R^3$ = Alkyl, (gegebenenfalls substituiertes) Aralkyl, Alkenyl, Alkinyl
$R^4$ = Alkyl, Aralkyl, Aryl
$R^5$ = Alkyl, Aralkyl, Aryl oder
$R^4$ und $R^5$ zusammen mit dem Kohlenstoffatom einen (gegebenenfalls substituierten oder überbrückten) carbocyclischen 5-, 6- oder 7-Ring
bedeuten,
als radikalische Initiatoren für die Herstellung von Polymerisaten auf der Basis polymerisierbarer α,β-ungesättigter Verbindungen.

Die Verbindungen der Formel I stellen 2,3-Dihydro-[1,2,4]triazolo[1,5-c]pyrimidine dar:

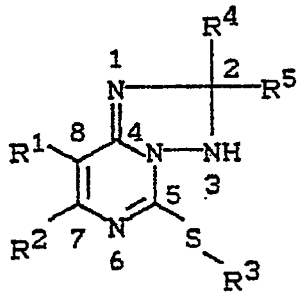

I

Besonders bevorzugt sind Verbindungen mit
$R^1$ = CN, Alkylsulfonyl,
$R^2$ = H, Alkyl, insbesondere $C_{1-8}$-Alkyl,
$R^3$ = $C_{1-8}$-Alkyl, $C_{7-13}$-Aralkyl, Alkenyl, insbesondere Propenyl, Allyl,
$R^4$ = $C_1$-$C_9$-Alkyl,
$R^5$ = $C_{1-9}$-Alkyl,
und $R^4$ und $R^5$ zusammen $(CH_2)_n$,
mit n = 4 oder 5.

Die Verbindungen der Formel I können hergestellt werden, indem man ein Acrylnitril der Formel II

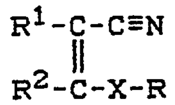

II,

in welcher
$R^1$ und $R^2$ die oben angegebenen Bedeutungen haben und R für H oder Alkyl sowie X für O oder S steht,
mit einem Isothiosemicarbazon der Formel III

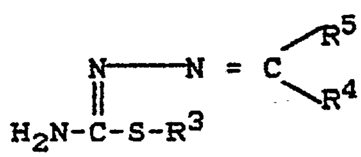

III,

in welcher
$R^3$, $R^4$ und $R^5$ die oben angegebenen Bedeutungen haben,
gegebenenfalls in Gegenwart eines Verdünnungsmittels umsetzt.

Im einzelnen seien die folgenden 2,3-Dihydro[1,2,4]triazolo[1,5-c]pyrimidine I genannt:

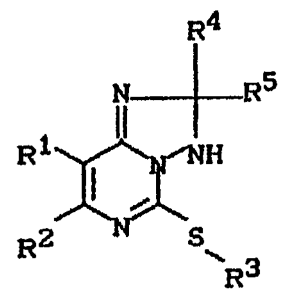

I

| Vbdg. Nr. | $R^1$ | $R^2$ | $R^3$ | $R^4$ | $R^5$ | Fp(° C) |
|---|---|---|---|---|---|---|
| I. 1 | NC | H | $CH_3$ | $CH_3$ | $CH_3$ | 176-179 (Z) |
| 2 | NC | $CH_3S$ | $CH_3$ | $-(CH_2)_5-$ | | 179-186 (Z) |
| 3 | NC | H | $CH_3$ | $-(CH_2)_4-$ | | 163-168 (Z) |
| 4 | NC | H | $CH_3$ | $-(CH_2)_5-$ | | 192-198 (Z) |
| 5 | $CH_3SO_2$ | H | $CH_3$ | $-(CH_2)_4-$ | | 140-143 (Z) |
| 6 | NC | H | $CH_3$ | $CH_3$ | $C(CH_3)_3$ | 185-186 (Z) |
| 7 | NC | H | $CH_3$ | $CH_3$ | $CH_2-CH(CH_3)_2$ | 128-130 (Z) |
| 8 | NC | H | $CH_3$ | $CH_3$ | $CH_2-C_6H_5$ | 184-186 (Z) |
| 9 | NC | H | $CH_2-C_6H_5$ | $CH_3$ | $CH_2-CH(CH_3)_2$ | 107-109 (Z) |
| 10 | NC | H | $CH_2-C_6H_5$ | $CH_3$ | $C(CH_3)_3$ | 124-127 (Z) |
| 11 | NC | H | $CH_2-C_6H_5$ | $CH_3$ | $C_6H_5$ | 121-126 (Z) |
| 12 | NC | H | $C_2H_5$ | $-(CH_2)_4-$ | | 134-138 |
| 13 | NC | H | $CH_2-C_6H_5$ | $-(CH_2)_4-$ | | 91- 93 (Z) |
| 14 | NC | H | $C_2H_5$ | $-(CH_2)_5-$ | | 162-168 (Z) |
| 15 | NC | H | $CH_3$ | $-CH(CH_3)-(CH_2)_4-$ | | 165-170 |

Fortsetzung

| Vbdg. | $R^1$ | $R^2$ | $R^3$ | $R^4$ | $R^5$ | Fp(°C) |
|---|---|---|---|---|---|---|
| 16 | NC | H | $CH_2-CH=CH_2$ | $-CH(CH_3)-(CH_2)_4-$ | | 122-127 (Z) |
| 17 | NC | H | $CH_2-C_6H_5$ | $-CH(CH_3)-(CH_2)_4-$ | | 135-138 (Z) |
| 18 | NC | H | $CH_3$ | $-CH_2-C(CH_3)_2-CH_2-CH(CH_3)-CH_2$ | | 129-132 (Z) |
| 19 | NC | H | $CH_3$ | $-C_6H_4(o)-(CH_2)_3-$ | | 218 (Z) |
| 20 | NC | H | $CH_2-CH=CH_2$ | $-C_6H_4(o)-(CH_2)_3-$ | | 150-152 |
| 21 | NC | H | $CH_2-C_6H_5$ | $-C_6H_4(o)-(CH_2)_3-$ | | 168-170 |
| 22 | NC | H | $CH_3$ | $-C_6H_4(o)-(o)C_6H_4-$ | | 229-232 (Z) |
| 23 | NC | $CH_3$ | $CH_3$ | $CH_3$ | $CH_3$ | 172-176 (Z) |
| 24 | NC | $CH_3S$ | $CH_3$ | $CH_3$ | $CH_3$ | 185-187 (Z) |
| 25 | NC | $CH_3S$ | $CH_3$ | $-(CH_2)_4-$ | | 140-144 (Z) |
| 26 | $C_6H_5$ | H | $C_2H_5$ | $CH_3$ | $CH_3$ | 155-158 |
| 27 | $CH_3SO_2$ | H | $CH_3$ | $CH_3$ | $CH_3$ | 173-175 (Z) |
| 28 | $(CH_3)_3C-CH_2-SO_2$ | H | $CH_3$ | $CH_3$ | $CH_3$ | 160-163 (Z) |
| 29 | $(CH_3)_3C-CH_2-SO_2$ | H | $C_2H_5$ | $CH_3$ | $CH_3$ | 159-160 (Z) |
| 30 | $(CH_3)_3C-CH_2-SO_2$ | H | $CH_3$ | $-(CH_2)_4-$ | | 122-124 (Z) |
| 31 | $(CH_3)_3C-CH_2-SO_2$ | H | $CH_3$ | $CH_3$ | $C(CH_3)_3$ | 152-156 (Z) |
| 32 | $(CH_3)_3C-C_6H_4(p)SO_2$ | H | $CH_3$ | $CH_3$ | $CH_3$ | 189-190 (Z) |
| 33 | $(CH_3)_3C-C_6H_4(p)SO_2$ | H | $C_2H_5$ | $CH_3$ | $CH_3$ | 155-157 (Z) |
| 34 | $(CH_3)_3C-C_6H_4(p)SO_2$ | H | $C_2H_5$ | $-(CH_2)_4-$ | | 161-162 (Z) |
| 35 | NC | H | $C_2H_5$ | $CH_3$ | $CH_3$ | 167-170 (Z) |

Verbindung I.1 ist bekannt [C. Yamazaki, Bull. Chem. Soc. Jpn. 54, 1167-1772 (1982)]. Die übrigen Verbindungen I.2 bis I.35 sind noch nicht bekannt. Verwendungsmöglichkeiten für derartige 2,3-Dihydro[1,2,4]triazolo[1,5-c]pyrimidine sind nicht vorbeschrieben.

Die Ausgangsprodukte der Formel II sind z. T. bekannt. Die neuen Verbindungen, z. B. die 1-Sulfonylacrylnitrile können hergestellt werden, indem man Mercaptane bzw. Thiophenole IV mit Chloracetonitril zu Alkyl- bzw. Aryl-cyanmethyl-thioethern V umsetzt, sie zu den entsprechenden Sulfonen VI oxydiert und durch Reaktion mit Orthoestern die Acrylnitrile IIa herstellt.

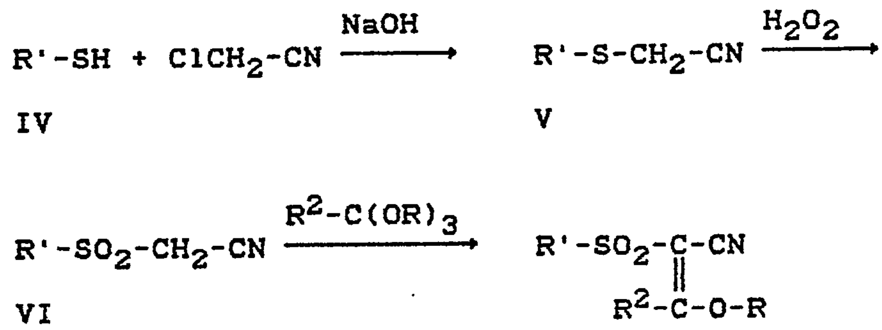


IV V VI IIa

Folgende Acrylnitrile der Formel II können z. B. eingesetzt werden:

| $R^1$ | $R^2$ | X | R | Fp(°C)[kp(°C)/mbar] |
|---|---|---|---|---|
| NC | H | O | $C_2H_5$ | 65 [91/0,15] |
| NC | $CH_3S$ | S | $CH_3$ | 81–82 |
| $CH_3SO_2$ | H | O | $CH_3$ | 112–115 |
| NC | $CH_3$ | O | $CH_3$ | [83/0,1] |
| $C_6H_5$ | H | O | H | 159–160 |
| $(CH_3)_3C–CH_2–SO_2$ | H | O | $CH_3$ | 79–80 |
| $(CH_3)_3C–C_6H_4(p)SO_2$ | H | O | $CH_3$ | 119–121° |

Die Ausgangsstoffe der Formel III sind teilweise bekannt. Die noch nicht bekannten Isothiosemicarbazone III können hergestellt werden, indem man ein Thiosemicarbazon VII mit einem Alkylierungsmittel zum Isothiosemicarbazonium-Salz VIII umsetzt und dieses mit Alkalihydroxid in die freie Base III überführt:

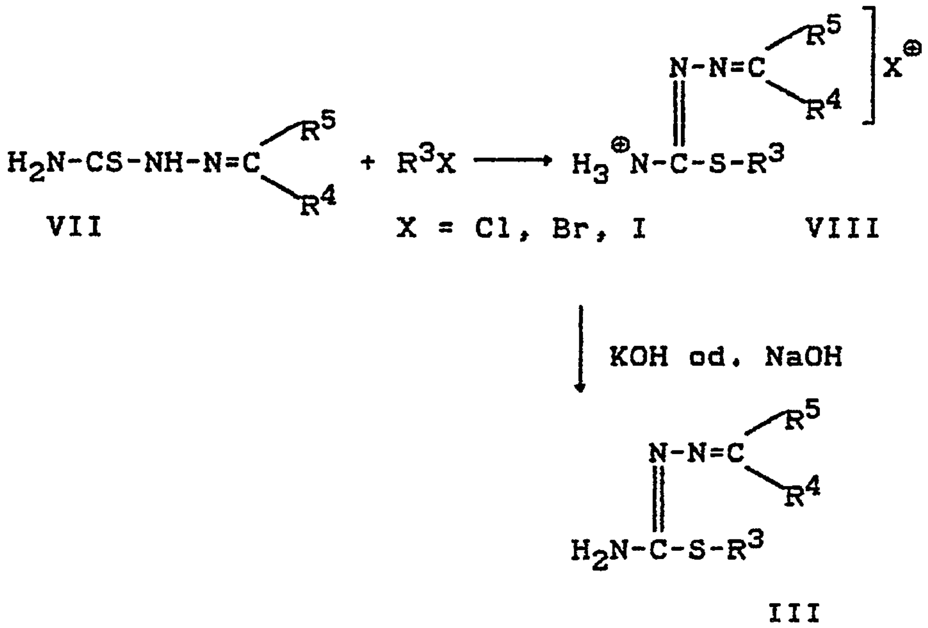

VII VIII III

Es können z. B. folgende Isothiosemicarbazone der Formel III eingesetzt werden:

| $R^3$ | $R^4$ | $R^5$ | Fp (°C) |
|---|---|---|---|
| $CH_3$ | $CH_3$ | $CH_3$ | 59–62 |
| $C_2H_5$ | $CH_3$ | $CH_3$ | 55–56 |
| $CH_3$ | | $-(CH_2)_5-$ | 76–77 |
| $CH_3$ | | $-(CH_2)_4-$ | 61–63 |
| $CH_3$ | $CH_3$ | $C(CH_3)_3$ | 57–58 |
| $CH_3$ | $CH_3$ | $CH_2-CH(CH_3)_2$ | 28–29 |
| $CH_3$ | $CH_3$ | $CH_2-C_6H_5$ | 39–41 |
| $CH_2-C_6H_5$ | $CH_3$ | $CH_2-CH(CH_3)_2$ | Oel |
| $CH_2-C_6H_5$ | $CH_3$ | $C(CH_3)_3$ | Oel |
| $CH_2-C_6H_5$ | $CH_3$ | C6H5 | 66–68 |
| $C_2H_5$ | | $-(CH_2)_4-$ | 70–71 |
| $CH_2-C_6H_5$ | | $-(CH_2)_4-$ | 70–71 |
| $C_2H_5$ | | $-(CH_2)_5-$ | 61–63 |
| $CH_3$ | | $-CH(CH_3)-(CH_2)_4-$ | 96–97 |
| $CH_2-CH=CH_2$ | | $-CH(CH_3)-(CH_2)_4-$ | Oel |
| $CH_2-C_6H_5$ | | $-CH(CH_3)-(CH_2)_4-$ | Oel |
| $CH_3$ | | $-CH_2-C(CH_3)_2-CH_2-CH(CH_3)-CH_2-$ | 71–72 |
| $CH_3$ | | $-C_6H_4(o)-(CH_2)_3-$ | 58–59 |
| $CH_2-CH=CH_2$ | | $-C_6H_4(o)-(CH_2)_3-$ | 58–60 |
| $CH_2-C_6H_5$ | | $-C_6H_4(o)-(CH_2)_3-$ | 53–55 |
| $CH_3$ | | $-C_6H_4(o)-(o)C_6H_4-$ | 84–87 |

Die Verbindungen I eignen sich für die radikalische Homopolymerisation oder Copolymerisation von Mischungen aus wenigstens zwei Monomeren des Typs, α,β-ungesättigte Verbindung.

Derartige Polymerisationen lassen sich in Substanz, Lösung, Emulsion, Suspension, Dispersion- oder Fällungspolymerisation durchführen.

Als Monomere geeignet sind radikalisch polymerisierbare Verbindungen, bevorzugt der Formel (II)

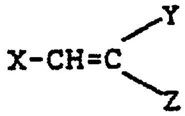

(II)

mit
X = H, $C_{1-4}$-Alkyl, $-CH=CH_2$ oder gemeinsam mit Z einen Cyclus bilden, insbesondere X = H,
Y = H, $C_{1-4}$-Alkyl, Aryl, COOX (mit X = H, $C_{1-4}$-Alkyl), CONHX (mit X = H, $C_{1-4}$-Alkyl), Halogen, -C≡N
Z = $C_{6-8}$-Aryl, $C_{1-14}$-Alkyl, substituiertes Aryl, Halogen, C≡N, COOX, CONX'X"
mit X und X" = H, Alkyl, Aryl, wobei die Alkyl- und Arylreste gegebenenfalls substituiert sein können.

Bevorzugte Verbindungen sind Vinyl-, Allyl- oder Maleinsäuremonomere. Insbesondere Styrol, α-Methylstyrol, p-Methylstyrol, Vinylcarbonatnaphthalin, Acrylnitril, Methacrylnitril, (Meth)-Acrylamide, (Meth)-Acrylsäureether, Vinylchlorid, -fluorid, Vinylidenhalogenide, Maleinsäureanhydrid, Maleinsäureimide, Vinylester von $C_{1-5}$-Carbonsäuren, Vinylether, Butadien, Isopren, Chloropren, cyclische Vinylverbindungen mit Amidstrukturen, Ethylen, Propylen, Buten sowie Monomere mit polyhalogenierten Seitenketten.

Die Initiatoren lassen sich bei Temperaturen von 25-150°C einsetzen, vorzugsweise im Bereich von 40-100°C. Dabei wird vorzugsweise mit Initiatorkonzentrationen von 0,01-3, vorzugsweise von 0,05-1,5 Gew.-%, bezogen auf 100 Teile zu polymerisierender Verbindung, gearbeitet.

Üblicherweise werden die Verbindungen (I) ähnlich wie bekannte Initiatoren verwendet, d.h. man arbeitet im günstigen Fall bei solchen Polymerisationstemperaturen, bei der der Initiator deutlich initiierend wirkt. Diese Temperaturen ergeben sich oftmals aus der Betrachtung der Halbwertszeiten.

Die Verbindungen (I) starten Polymerisation überraschenderweise gut in Gegenwart von atmosphärischer Luft; gegenüber Schwankungen der Luftkonzentration ist dabei der Start einer Polymerisation unempfindlich, d.h. Initiierungsverzögerungen, reduzierte Polymerisatausbeute sowie unkontrollierte Polymermolekulargewichte treten nicht ein.

Die gewonnenen Polymerisate weisen gute Eigenschaften und hohe Thermostabilitäten auf.

Beispiel 1

I. Verbindungen der Formel I (als Initiatoren)

I.1

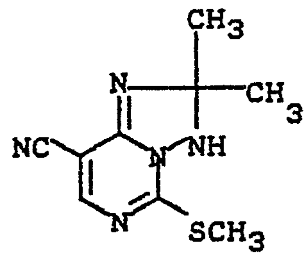

I.2

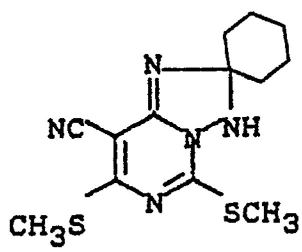

I.3

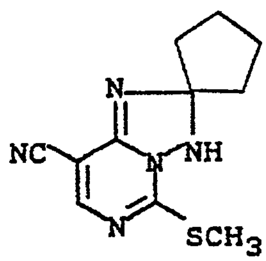

I.4

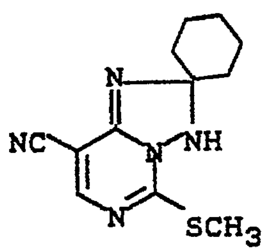

I.5

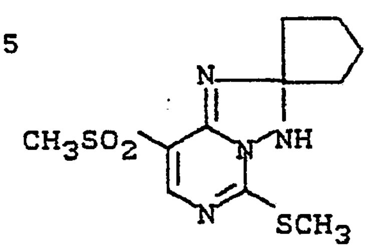

Zum Vergleich (als Initiator)
I.6 Cumolhydroperoxid

II. <u>Herstellung der Verbindungen</u>

I.1 bis I.5

I.1

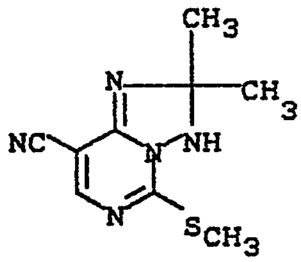

Eine Lösung von 70,8 g Ethoxymethylen-malodinitril (0,58 Mol) und 87,0 g Aceton-S-methyl-isothiosemicarbazon (0,6 Mol) in 1 l Toluol wird 4 Stunden unter Rückfluß gekocht. Nach Filtration läßt man bei 0°C kristallisieren und erhält 88,0 g (68,7 % d. Th.) gelbe Kristalle, die bei 176-179°C unter Zersetzung schmelzen.

I.2

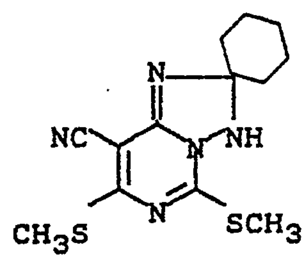

Eine Mischung von 68,0 g 1,1-Dicyan-2,2-dimethylmercaptoethylen (0,4 Mol) und 74 g Cyclohexanon-S-methyl-isothiosemicarbazon wird ohne Lösungsmittel unter Aufheizen gerührt. Ab 57°C entsteht eine homogene Schmelze, die sich ab 89°C wieder allmählich verfestigt. Man läßt ohne Rühren bei 100°C bis zum Ende der Methylmercaptan-Entwicklung (ca. 90 Minuten) ausreagieren, gibt 250 ml Acetonitril zu und kühlt auf 0°C. Nach Absaugen und mehrmaligem Waschen mit Acetonitril erhält man 81,0 g (66,0 % d. Th.) gelbe Kristalle vom Schmelzbereich 179-186°C (Zers.).

I.3

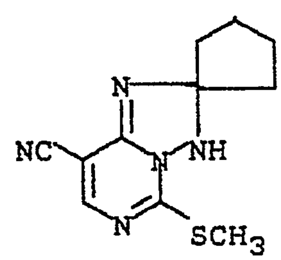

Man löst 238 g Ethoxymethylen-malodinitril (1,95 Mol) sowie 333,5 g Cyclopentanon-S-methyl-isothiosemicarbazon (1,95 Mol) in jeweils 1,95 l Toluol und vereinigt die Lösungen unter Rühren bei 20°C. Die Reaktion verläuft exotherm, innerhalb 1,5 Stunden steigt die Temperatur der Lösung auf 31°C, danach fällt das gelbe Reaktionsprodukt aus. Nach 2 Tagen wird abgesaugt, mit wenig Toluol und mit Petrolether gewaschen. Man erhält 362,2 g (75,2 % d. Th.) hellgelbe Kristalle vom Schmelzpunkt 163-168°C (Zers.).

I.4

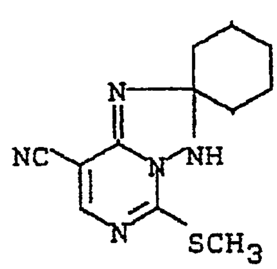

48,8 g Ethoxymethylen-malodinitril (0,4 Mol) sowie 74,0 g Cyclohexanon-S-methyl-isothiosemicarbazon (0,4 Mol) werden in jeweils 400 ml Toluol gelöst und analog Beispiel I.3 umgesetzt. Man erhält 74,4 g (71,3 % d. Th.) leuchtend gelbe Kristalle vom Schmelzpunkt 192-198°C (Zers.).

I.5

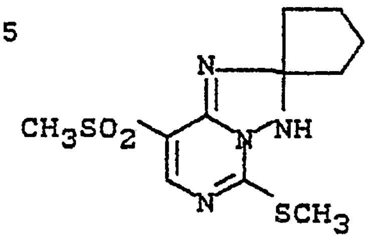

Eine Lösung von 17,1 g Cyclopentanon-S-methyl-isothiosemicarbazon (0,1 Mol) in 100 ml Toluol versetzt man mit 16,1 g 1-Methylsulfonyl-2-methoxy-acrylnitril (0,1 Mol) und heizt bis zur Lösung der zweiten Komponente auf 70°C. Nach Abklingen der schwach exothermen Reaktion rührt man kurz bei 85°C nach, saugt bei 20°C ab und wäscht mit Toluol. Man erhält 18,1 g (60,3 % d. Th.) hellgelbe Nadeln vom Schmelzpunkt 140-143°C (Zers.).

III. Durchführung der Polymerisation

50 Gew.-Teile Styrol werden in einem Reaktor mit 0,1 Gew.-Teilen der Verbindungen I1 bis I6 versetzt. Bei 100°C wird 2 Stunden unter Rühren polymerisiert. Danach wird abgekühlt und das Polymerisat mit Alkohol ausgefällt. Bestimmt wird der K-Wert der Polymerisate in Dimethylformamid bei 25°C. Die Versuche werden in Gegenwart von Luft durchgeführt.

Die Ergebnisse sind in Tabelle 1 zusammengefaßt.

Tabelle 1
Styrolpolymerisation

| Versuch | Polymerausbeute (Gew.-%) | K-Wert | eingesetzter Starter |
|---|---|---|---|
| III.1 | 25 | 46 | I.1 |
| III.2 | 23 | 52 | I.2 |
| III.3 | 26 | 55 | I.3 |
| III.4 | 20 | 49 | I.4 |
| III.5 | 24 | 50 | I.5 |
| III.6 | 23 | 49 | I.6 |
| III.7 | 4 | 82 | ohne Starterzusatz |
| III.8 | 20 | 49 | I.4*) |

*) Während der Polymerisation wurde stetig in Sauerstoffatmosphäre gearbeitet (Gasdurchleitung im Reaktor).

Beispiel 2

In einem Rundkolben, ausgestattet mit einer Rührvorrichtung, einem Temperaturmeßgerät, einem Gaseinleitungsrohr und einem Rückflußkühler, werden 5,0 Gew.-Teile Acrylnitril und 0,0125 Gew.-Teile der Verbindung I.4 (aus Beispiel 1) vorgelegt. Es wird ein schwacher Luftstrom eingeleitet. Nach Temperierung auf 77°C trübt sich das Reaktionsgemisch durch Ausscheidung von weißen Polymerisatpartikeln ein. 2,5 Stunden nach Beginn der Polymerisation wird das gebildete Polymerisat durch Eintragen in 100 Vol.-Teile Methanol gereinigt und danach isoliert.
Ausbeute: 1,2 Gew.-Teile (Umsatz: 24 %)
$\eta_{rel}$ = 6,88 (gemessen an einer 0,5 gew.-%igen Lösung in Dimethylformamid)

Unter vergleichbaren Bedingungen wird ohne Zusatz der Verbindung I.4 keine Polymerisation beobachtet.

Beispiel 3

In einem Rundkolben, ausgestattet mit einer Rühvorrichtung, einem Temperaturmeßgerät, einem Gaseinleitungsrohr und einem Rückflußkühler, werden 5,0 Gew.-Teile N-Phenylmaleinimid (Fp. 89°C) und 0,0125 Gew.-Teile der Verbindung I.4 (aus Beispiel 1) vorgelegt. Es wird ein schwacher Luftstrom eingeleitet. Nach Aufschmelzen des Monomeren und Temperierung des Reaktionsgemisches auf 90°C setzt

die Polymersation sofort ein, erkenntlich an der Ausscheidung eines gelblich-weißen Polymerisatpulvers. Nach 2,5 Stunden wird das gebildete Poly-N-Phenylmaleinimid durch Einrühren in 100 Vol.-Teile Methanol isoliert.
Ausbeute: 1,4 Gew.-Teile (Umsatz: 28 %)
$\eta_{rel.}$ = 1,17 (gemessen an einer 0,5 gew.-%igen Lösung in Dimethylformamid)

Unter vergleichbaren Bedingungen wird bei Fehlen der Verbindung I.4 keine Polymerisation beobachtet.

Beispiel 4 (Lösungspolymerisation)

1. Lösungsmittel Methylethylketon

Ein Gemisch aus 30 Gew.-Teilen Methylmethacrylat und 10 Gew.-Teilen eines Perfluoralkylmethacrylates der Formel

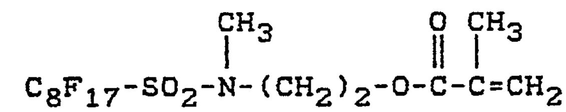

wird mit 60 Gew.-Teilen Methylethylketon gemischt und 4 Stunden bei 80° C gehalten, nachdem als Starter 0,4 Gew.-Teile der Verbindung I.4 aus Beispiel 1 in die Reaktionsmischung gegeben wurde.

Der Versuch wird wiederholt, aber ohne Zugabe der Verbindung I.4.

Die aus beiden Versuchen resultierenden Polymerisate werden durch Eintragen des Reaktionsgemisches in Methanol ausgefällt, einmal mit etwa 400 Gew.-Teilen Methanol gekocht und im Vakuumschrank bei 70°C getrocknet. Mit Verbindung I.4 erhält man 12,4 Gew.-Teile Copolymerisat, das Perfluoralkylmethacrylat enthält, ohne Verbindung I.4 4,5 Gew.-Teile Copolymerisat, das Perfluoralkylmethacrylat enthält. Die fast um den Faktor 3 höhere Polymerisatausbeute zeigt die Wirksamkeit der anmeldungsgemäßen Starter.

2. Emulsionspolymerisation

In einem Polymerisationsgefäß werden 300 Gew.-Teile Wasser, 35 Gew.-Teile Butylmethacrylat, 35 Gew.-Teile Ethylmethacrylat, 0,35 Gew.-Teile der Verbindung I.4 aus Beispiel 1 und 2,1 Gew.-Teile eines anionischen Netzmittels (Hostapal BV) mit einem Schnellrührer emulgiert. Man hält die entstehende Dispersion 3 Stunden auf 100°C. Es resultiert eine Polymerisatdispersion mit einem Feststoffgehalt von 17,9 %. Das entspricht einem Umsatz bei der Polymerisation von >95 %.

## Patentansprüche

1. 2,3-Dihydro[1,2,4]triazolo[1,5-c]pyrimidine der Formel I

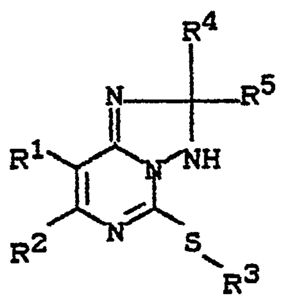

I

in welcher
$R^1$ = CN, Alkylsulfonyl, Arylsulfonyl, Aryl,
$R^2$ = H, Alkyl, Alkylthio
$R^3$ = Alkyl, (gegebenenfalls substituiertes) Aralkyl, Alkenyl, Alkinyl
$R^4$ = Alkyl, Aralkyl, Aryl
$R^5$ = Alkyl, Aralkyl, Aryl oder
$R^4$ und $R^5$ zusammen mit dem Kohlenstoffatom einen (gegebenenfalls substituierten oder überbrückten) carbocyclischen 5-, 6- oder 7-Ring bedeuten,
worin $R^4$ nicht Methyl oder Aryl sein kann, wenn $R^1$ CN, $R^2$ H, $R^3$ Methyl und $R^5$ Methyl oder Aryl ist.

2. Verfahren zur Herstellung der Verbindungen gemäß Anspruch 1, dadurch gekennzeichnet, daß

man ein Acrylnitril der Formel II

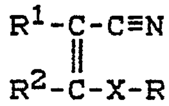

II,

in welcher
$R^1$ und $R^2$ die oben genannten Bedeutungen haben und R für H oder Alkyl sowie X für O oder S steht, mit einem Isothiosemicarbazon der Formel III

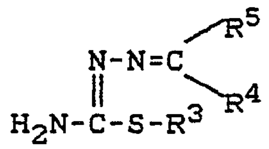

III,

in welcher
$R^3$, $R^4$ und $R^5$ die oben angegebenen Bedeutungen haben, gegebenenfalls in Gegenwart eines Verdünnungsmittels umsetzt.

3. Verwendung von Verbindungen der Formel

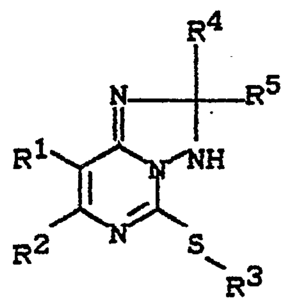

I

in welcher
$R^1$ = CN, Alkylsulfonyl, Arylsulfonyl, Aryl,
$R^2$ = H, Alkyl, Alkylthio
$R^3$ = Alkyl, (gegebenenfalls substituiertes) Aralkyl, Alkenyl, Aralkyl, Alkinyl
$R^4$ = Alkyl, Aralkyl, Aryl
$R^5$ = Alkyl, Aralkyl, Aryl oder
$R^4$ und $R^5$ zusammen mit dem Kohlenstoffatom einen (gegebenenfalls substituierten oder überbrückten) carbocyclischen 5-, 6- oder 7-Ring bedeuten,
als Initiatoren für die radikalische Polymerisation.

**Claims**

1. 2,3-Dihydro[1, 2, 4]triazolo[1,5-c]pyrimidines of the formula I

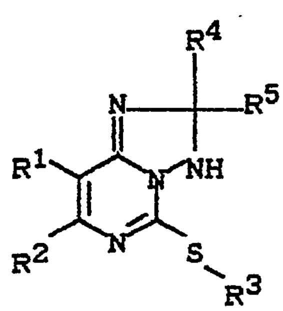

I

in which
$R^1$ = CN, alkylsulphonyl, arylsulphonyl or aryl,
$R^2$ = H, alkyl or alkylthio
$R^3$ = alkyl, (optionally substituted) aralkyl, alkenyl or alkinyl
$R^4$ = alkyl, aralkyl or aryl
$R^5$ = alkyl, aralkyl, aryl or
$R^4$ and $R^5$, together with the carbon atom, denote an (optionally substituted or bridged) carbocyclic 5-,

6- or 7-membered ring,

where $R^4$ cannot be methyl or aryl if $R^1$ is CN, $R^2$ is H, $R^3$ is methyl and $R^5$ is methyl or aryl.

2. Process for the preparation of compounds according to Claim 1, characterized in that an acrylonitrile of the formula II

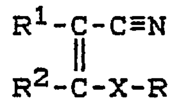 II,

in which

$R^1$ and $R^2$ have the meanings given above and R represents H or alkyl, and X represents O or S, is reacted, optionally in the presence of a diluent, with an isothiosemicarbazone of the formula III

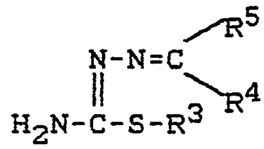 III,

in which

$R^3$, $R^4$ and $R^5$ have the meanings given above.

3. The use of compounds of the formula

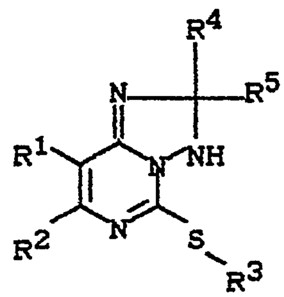 I

in which

$R^1$ = CN, alkylsulphonyl, arylsulphonyl or aryl,

$R^2$ = H, alkyl or alkylthio

$R^3$ = alkyl, (optionally substituted) aralkyl, alkenyl, aralkyl or alkinyl

$R^4$ = alkyl, aralkyl or aryl

$R^5$ = alkyl, aralkyl, aryl or

$R^4$ and $R^5$, together with the carbon atom, denote an (optionally substituted or bridged) carbocyclic 5-, 6- or 7-membered ring,

as initiators for free-radical polymerization.

## Revendications

1. 2,3-dihydro[1, 2, 4]triazolo[1,5-c]pyrimidines de formule I

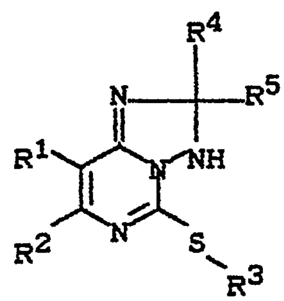 I

dans laquelle

$R^1$ est un groupe CN, alkylsulfonyle, arylsulfonyle, aryle,

$R^2$ représente H, un groupe alkyle, alkylthio

$R^3$ est un groupe alkyle, aralkyl (éventuellement substitué), alcényle, alcynyle

$R^4$ est un groupe alkyle, aralkyle, aryle

$R^5$ est un groupe alkyle, aralkyle, aryle, ou bien

$R^4$ et $R^5$ forment, conjointement avec l'atome de carbone, un noyau carbocylique pentagonal, hexagonal ou heptagonal (éventuellement substitué ou ponté),

$R^4$ ne pouvant pas être un groupe méthyle ou aryle lorsque $R^1$ est un groupe CN, $R^2$ représente H, $R^3$ est un groupe méthyle et $R^5$ est un groupe méthyle ou aryle.

2. Procédé de production des composés suivant la revendication 1, caractérisé en ce qu'on fait réagir un acrylonitrile de formule II

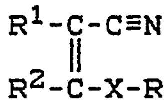 II,

dans laquelle

$R^1$ et $R^2$ ont les définitions indiquées ci-dessus et R est l'hydrogène ou un groupe alkyle, de même que X représente O ou S,

avec une isothiosemicarbazone de formule III

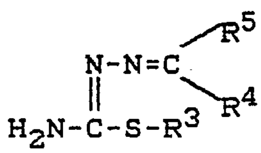 III,

dans laquelle

$R^3$, $R^4$ et $R^5$ ont les définitions indiquées ci-dessus, le cas échéant en présence d'un diluant.

3. Utilisation de composés de formule

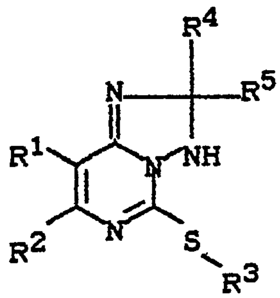 I

dans laquelle

$R^1$ est un groupe CN, alkylsulfonyle, arylsulfonyle, aryle,

$R^2$ est l'hydrogène, un groupe alkyle, alkylthio

$R^3$ est un groupe alkyle, un groupe aralkyle (éventuellement substitué), alcényle, aralkyle, alcynyle

$R^4$ est un groupe alkyle, aralkyle, aryle

$R^5$ est un groupe alkyle, aralkyle, aryle, ou bien $R^4$ et $R^5$ forment conjointement avec l'atome de carbone un noyau carboxyclique pentagonal, hexagonal ou heptagonal (éventuellement substitué ou ponté),

comme initiateurs de polymérisation radicalaire.